# EUROPEAN PATENT APPLICATION

(11) **EP 2 992 845 A1**
(43) Date of publication of application: **09.03.2016**
(21) Application number: 15188588.6
(22) Date of filing: 30.06.2011
(51) Int. Cl.: A61B 17/70

(54) **SPINAL STABILIZATION SYSTEM UTILIZING SCREW AND EXTERNAL FACET AND/OR LAMINA FIXATION**

(30) Priority: 08.07.2010 US 362334 P
(62) Divisional of application: 11732538.1
(71) Applicant: X-spine Systems, Inc., Miamisburg, OH 45342 (US)
(72) Inventor: Kirschmann, Louis David, Dayton, OH 45429 (US)
(74) Representative: Kessler, Stephan

(57) **Abstract**

A surgical implant for support of spinal vertebrae comprising a screw element having a threaded shank for screwing into a pedicle of a first vertebra and a fixation component that is moveably secured to the screw element. The fixation component has a first end for receiving the screw element and a fixation surface adapted to engage or attach to at least one of an external facet or laminar surface of a second vertebra. Various embodiments are provided for polyaxial movement of the fixation component relative to a polyaxial screw. Also, various apparatus and means for locking the screw element to the fixation component are provided.

## Description

### CROSS-REFERENCE TO RELATED APPLICATION

The present application claims priority to provisional U.S. Application Serial No. 61/362,334 filed July 8, 2010, and to U.S. Patent Application Serial No. 13/173,660, filed June 30, 2011, to which Applicant claims the benefit of the earlier filing dates. These applications are incorporated herein by reference and made a part hereof.

### BACKGROUND OF THE INVENTION

### 1. Field of the Invention

This invention relates to a spinal implant and, more particularly, to a spinal implant having a screw and a fixation component and variations thereof.

### 2. Description of the Related Art

The field of spinal implantation burgeons with devices and methods for the achievement of fixation between adjacent vertebrae. The most common devices currently used for fixation are pedicle screw systems. In a typical pedicle screw system, screws are placed into pedicles of adjacent vertebrae and are stabilized together using various separate rod or plate means. An emerging means for achieving fixation of adjacent vertebra is the use of trans-facet fixation. Several devices achieve fixation by placement of a screw or other means through the facet joint. This procedure has the advantage of being significantly less invasive than pedicle screw procedures because it does not require a separate rod or plate means and only requires two bilateral screws to achieve fixation per level, rather than four in a typical pedicle screw system. For these reasons, trans-facet fixation has been growing in popularity.

Some of the systems for bone fixation relating to facet fusion are shown or known from U.S. Patent Publications Nos. 2010/0114175 to McKay; 2010/0100135 to Phan; 2010/0094356 to Varela et al.; 2010/0087859 to Jackson, Jr.; 2010/0082065 to Butler et al.; 2010/0076490 to Greenwald et al.; 2009/0318980 to Falahee; 2009/0312763 to McCormack et al.; 2009/0312798 to Varela; 2009/0312800 to Chin et al.; 2009/0306671 to McCormack et al.; 2009/0299412 to Marino; 2009/0275954 to Phan et al.; 2009/0275992 to Phan et al.; 2009/0275993 to Phan et al.; 2009/0275994 to Phan et al.; 2009/0270929 to Suddaby; 2009/0264928 to Blain; 2009/0248082 to Crook et al.; 2009/0248089 to Jacofsky et al.; 2009/0234394 to Crook; 2009/0234397 to Petersen; 2009/0216273 to Cox; 2009/0192551 to Cianfrani et al.; 2009/0187219 to Pachtman et al.; 2009/0177205 to McCormack et al.; 2009/0163920 to Hochschuler et al.; 2009/0131986 to Lee et al.; 2009/0125066 to Kraus et al.; 2009/0112264 to Lins; 2009/0105819 to Barry; 2009/0099602 to Aflatoon; 2009/0093851 to Osman; 2009/0076551 to Petersen; 2009/0054903 to Falahee et al.; 2009/0036926 to Hestad; 2009/0036927 to Vestgaarden; 2009/0036986 to Lancial et al.; 2008/0275454 to Geibel; 2008/0262555 to Assell et al.; 2008/0255618 to Fisher et al.; 2008/0255619 to Schneiderman et al.; 2008/0255622 to Mickiewicz et al.; 2008/0255666 to Fisher et al.; 2008/0255667 to Horton; 2008/0234758 to Fisher et al.; 2007/0233092 to Falahee; 2007/0233093 to Falahee; 2007/0112428 to Lancial; 2006/0264953 to Falahee; 2006/0212034 to Triplett et al.; 2006/0200149 to Hoy et al.; 2006/0111779 to Petersen; 2006/0111780 to Petersen; 2005/0267480 to Suddaby; 2005/0149030 to Serhan et al.; 2005/0124993 to Chappuis; 2004/0254575 to Obenchain et al.; 2004/0087948 to Suddaby; and 2003/0208202 to Falahee, all of which are incorporated herein by reference and made a part hereof. Other systems are shown in U.S. Patent Nos. 6,648,893 issued to Dudasik; 7,608,094 issued to Falahee; 7,708,761 issued to Petersen; 7,563,275 issued to Falahee et al.; 7,699,878 issued to Pavlov et al.; 7,452,369 issued to Barry; 7,223,269 issued to Chappuis; 6,540,747 issued to Marino; 6,485,518 issued to Cornwall et al., all of which are incorporated herein by reference and made a part hereof.

There are significant disadvantages, however, to trans-facet fixation. The procedure is technically demanding, particularly in the so-called translaminar approach where the fixation screw must be placed very close to critical nerve elements. Compared to pedicle anatomy, which is relatively constant from patient-to-patient, facet anatomy is quite variable and can lead to variations in screw purchase and fixation strength. Furthermore, the facet joints are rather small compared to the pedicles are far more prone to fracturing than pedicles during screw placement.

Therefore, what is needed is a new apparatus, system and method that preserves the minimally-invasive nature of trans-facet fixation while mitigating the problems of anatomy variation, facet fracture, and technical difficulty.

### SUMMARY OF THE INVENTION

The facet joint has two general parts, the internal articular surface which functions to slide against the adjacent facet joint of an adjacent vertebra, and a bony external surface which arises from the lamina or posterior portion of the vertebrae. This external surface is comprised of cortical bone. This external portion is strong, easily accessible, and safely away from critical nerve elements. It is the intention of one embodiment to use this surface as a fixation point. It is further the intention of one embodiment to use the pedicle of the adjacent vertebra as the other fixation point.

One object of one embodiment is to provide an improved spinal stabilization system utilizing screw and external facet and/or laminar fixation.

Another object of another embodiment is to provide a fixation component that can be removably mounted on a screw.

Still another object of another embodiment is to provide a surgical implant having a fixation component that is fixed relative to the screw.

Still another object of another embodiment is to provide a fixation component that can move polyaxially relative to the screw.

Still another object is to provide various apparatus and means for securing the fixation component to the screw and for providing polyaxial movement of the fixation component relative to the screw.

In one aspect, one embodiment of the invention provides a surgical implant for support of spinal vertebrae, the surgical implant comprising a screw element having a threaded shank for screwing into a pedicle of a first vertebra, a fixation component that is moveably secured to the screw element, the fixation component having a first end for receiving the screw element and a fixation surface adapted to engage or attach to at least one of an external facet or laminar surface of a second vertebrae.

In another aspect, another embodiment of the invention provides a fixation component that is moveably secured to a screw element, the fixation component comprising, a first end secured to the screw element and a fixation surface adapted to engage or attach to at least one of an external facet or laminar surface of a second vertebrae.

These and other objects and advantages will be apparent from the following description, the accompanying drawings and the appended claims.

### BRIEF DESCRIPTION OF THE DRAWINGS

Fig. 1 is a view of one embodiment showing a fixation component attached to a screw element;
Fig. 2 is an exploded view of the embodiment shown in Fig. 1;
Fig. 3 is a view showing various details of the screw element;
Fig. 4 is a view of the fixation component shown in Fig. 1 showing an angled profile and plurality of engagement teeth;
Fig. 5 is a plan view of the fixation component shown in Fig. 1;
Fig. 6 is a view of another embodiment of the fixation component showing an elongate portion having a curved fixation surface;
Fig. 7 is a view showing further details of the fixation component shown in Fig. 6;
Fig. 8A is a view of another embodiment illustrating a fixation component that cooperates with a split ball and locking nut having a inner spherical surface to permit polyaxial movement of the fixation component;
Fig. 8B is a sectional view taken along the line 8B-8B in Fig. 8A;
Fig. 9 is an exploded view of the embodiment shown in Figs. 8A and 8B;
Figs. 10A-10F are various embodiments illustrating polyaxial movement of the fixation component relative to a polyaxial screw used with or without a retainer or housing and with various means for fixing or locking the fixation component to the polyaxial screw;
Fig. 11 is a view illustrating a placement of the spinal implant to fix a facet joint; and
Figs. 12A-12B are further enlarged fragmentary views of a portion of the view in Fig. 11 further illustrating the use of the fixation component.

### DETAILED DESCRIPTION OF PREFERRED EMBODIMENTS

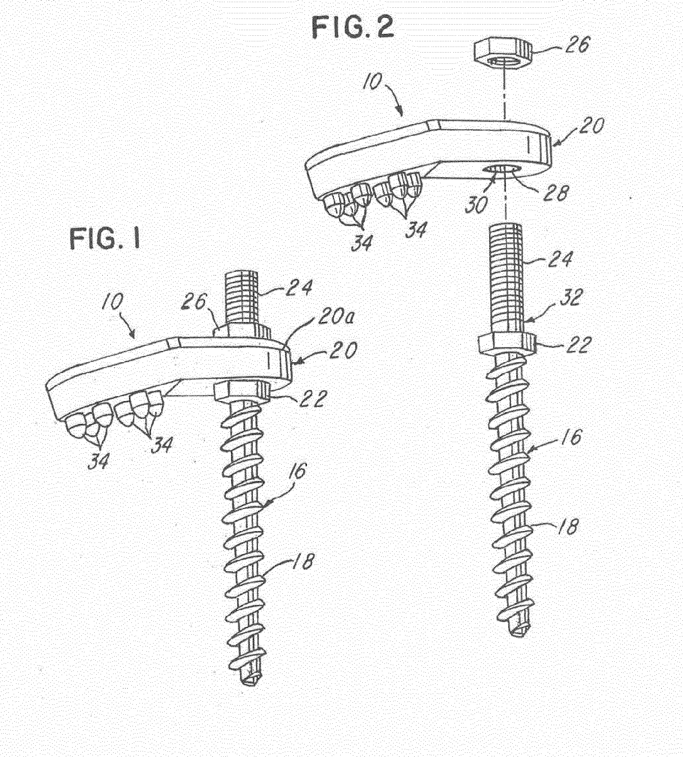

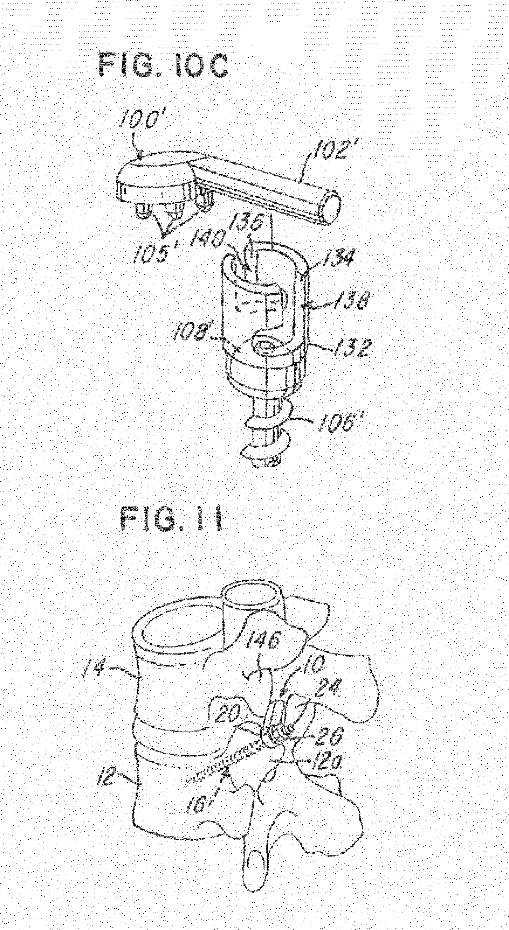

Referring now to Figs. 1-7, a surgical implant 10 is shown. In the illustration being described, the surgical implant 10 is for the support of spinal vertebrae, such as a first vertebra 12 (Fig. 11) and a second vertebra 14. The surgical implant 10 (Figs. 1-5) is adapted to be secured to, for example, the first vertebra 12 and adapted to engage or attach to at least one of an external facet or laminar surface 14b of the second vertebra 14.

In the illustration being described, the surgical implant 10 comprises a screw element 16 (Figs. 1-2) having a threaded shank 18 for screwing into a facet 12a (Fig. 11) of the first vertebra 12. The surgical implant 10 further comprises a fixation component 20 that is moveably secured to the screw element 16. The fixation component 20 comprises a first end 20a and a second end 20b.

The screw element 16 comprises a nut 22 that is integrally formed therewith and a threaded portion 24 that threadably receives a nut 26 best shown in Figs. 1 and 2. Note that the fixation component 20 comprises an inner wall 28 that defines an aperture or bore 30 for receiving the threaded portion 24 as best illustrated in Fig. 2. In this regard, it should be understood that the inner wall 28 comprises a circumference that is slightly larger than the threaded portion 24 so that the threaded portion 24 can be inserted through the aperture 30.

The screw element 16 comprises a connection portion 32 which in the embodiment of Figs. 1 and 2 is defined by the threaded portion 24. In the illustration being described in Figs. 1 and 2, the threaded portion 24 is at an opposite end of the threaded shank 18 and is adapted and dimensioned to removably receive the fixation component 20. The threaded portion 24 is coaxial with the threaded shank 18.

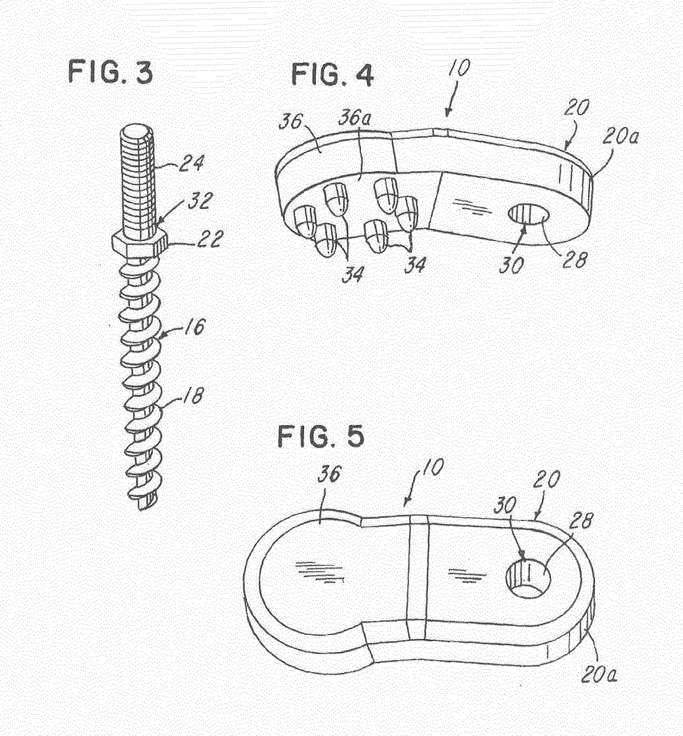

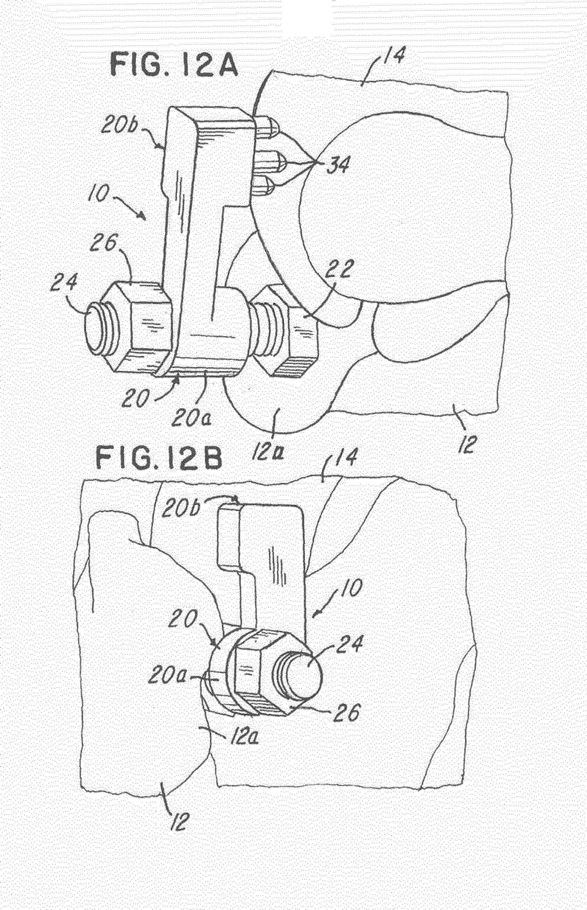

The second end 20b of the fixation component 20 has a fixation or frictional surface 36a of a body 36 that defines the fixation component 20. The plurality of engagement teeth 34 are adapted to engage or attach to at least one of the external facet or laminar surface 14b of the second vertebra 14. In regards to the engagement teeth 34 (Figs. 1 and 4), it should be understood that fewer or more teeth may be used, or alternatively, a serrated or other type of machined surface on the frictional surface 36a of the body 36 may be provided to facilitate frictional engagement of the bone. In the illustration being described, it should be understood that the second vertebra 14 is immediately adjacent the first vertebra 12 as shown in Figs. 11 and 12A-12B; however, it should be understood that the fixation component 20 could be adapted to traverse a greater distance such that it can engage a vertebra (not shown) that is not immediately adjacent to the first vertebra 12.

Returning to the embodiment of Figs. 1-5, it should be understood that while the fixation component 20 is shown as having a body having a generally angled profile, the body could comprise a linear shape, a curvo-linear shape, a concave shape, semi-spherical shape, multi-angular shape or other shape or surface to generally correspond to or match an anatomy of the at least one of the external facet or laminar surface 14b of the second vertebra 14.

After the surgeon screws the screw element 16 into the facet 12a of the first vertebra 12, as shown in Figs. 11 and 12A-12B, the fixation component 20 is mounted or guided onto the screw element 16 by inserting the threaded portion 24 through the aperture 30. The nut 26 is then threadably mounted on the threaded portion 24 to secure the fixation component 20 to the connection portion 32 of the screw element 16.

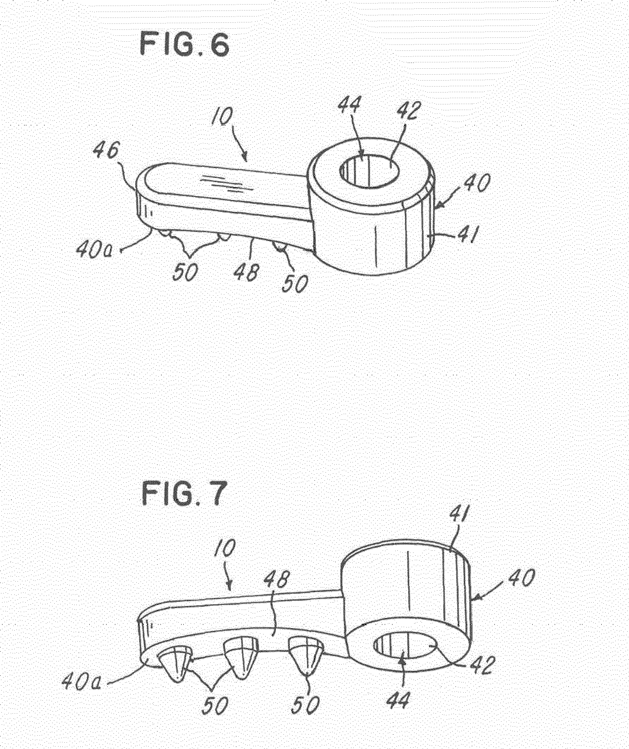

Figs. 6 and 7 illustrate another fixation component 40 that could be mounted on the screw element 16. In this embodiment, the fixation component 40 has a first portion 41 having an internal wall 42 that defines an aperture 44 for receiving the connection portion 32, which in the illustration in Figs. 1-5 is the threaded portion 24. Note that the fixation component 40 in the embodiment of Figs. 6 and 7 has an elongated portion 46 having a curved surface 48 that is adapted or shaped to match a desired anatomy. In this illustration, the elongated portion 46 extends generally radially from an axis defined by the aperture 44 and comprises a fixation surface 40a having a plurality of engagement teeth 50 that are aligned linearly. As with the embodiment of Figs. 1-5, the embodiment of the fixation component 40 in Figs. 6 and 7 would be mounted on the screw element 16 and secured thereto with the nut 26.

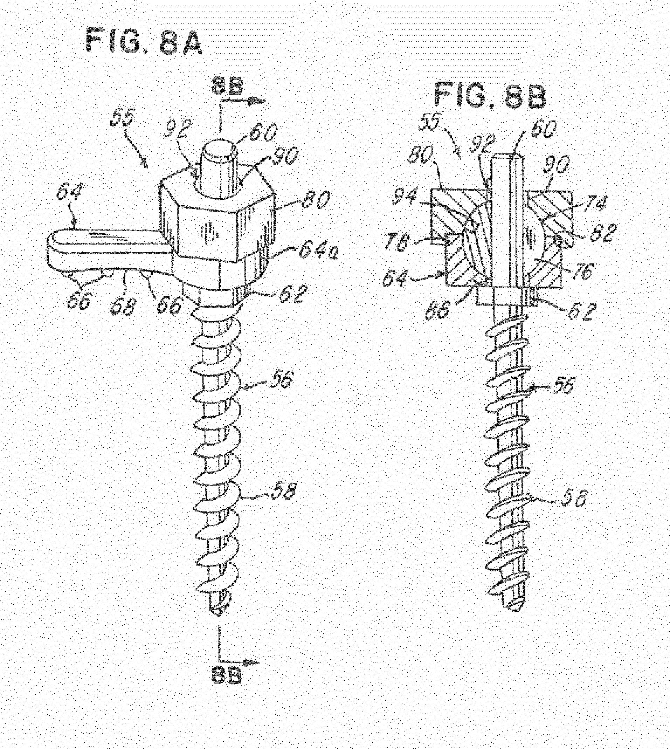

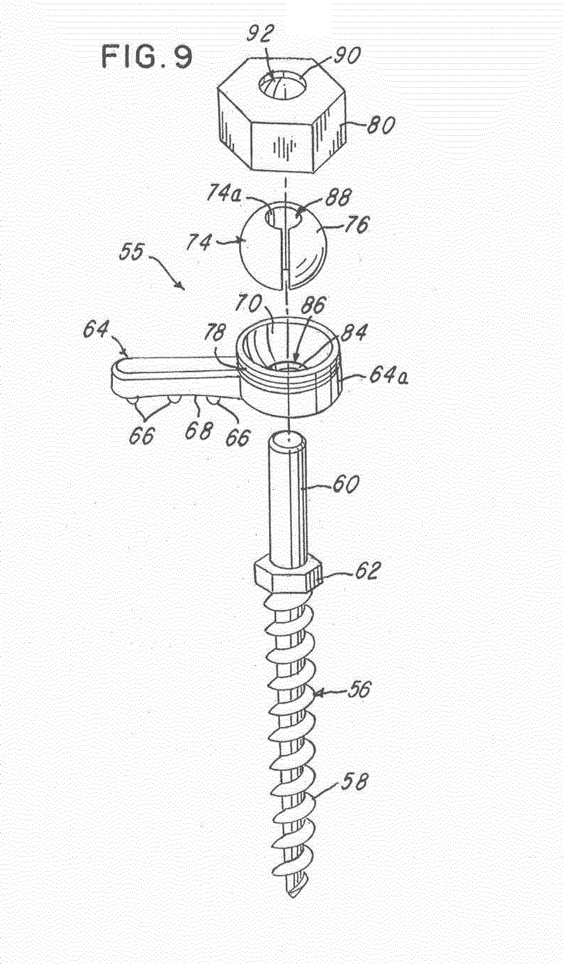

It may be desired to provide a fixation component that moves polyaxially relative to the screw element 16. Figs. 8A-10F illustrate various embodiments for permitting such polyaxial movement. Figs. 8A, 8B and 9 illustrate an embodiment of a surgical implant 55 that comprises a screw element 56 having a threaded shank 58 and a connection portion 60 that is generally cylindrical and coaxial with the threaded shank 58 as shown. As with the previous embodiments, the screw element 56 comprises an integral nut 62 for screwing the screw element 56 into bone.

In the illustration being described, the surgical implant 55 comprises a fixation component 64 having a plurality of teeth 66 that are integral with a curved surface 68 similar to the curved surface 48 in the embodiment shown in Figs. 6 and 7. Note that a first end 64a of the fixation component 64 comprises an interior spherical surface that is adapted and dimensioned to receive a compression element 74 in the form of a split ball 76. The first end 64a also comprises external or male threads 78 that are adapted to receive a nut 80 having female threads 82 (Fig. 8B) that threadably receive the male threads 78.

In the illustration being shown in Figs. 8A, 8B and 9, the fixation component 64 comprises an interior wall 84 defining a bore 86 for receiving the connection portion 60. After the screw element 56 is situated into bone, such as the first vertebra 12 in Fig. 11, the fixation component 64 (Fig. 9) receives the connection portion 60 which receives the split ball 76. In this regard, the split ball 76 has an interior wall 74a that defines a bore 88 that is adapted to receive the connection portion 60.

After the fixation component 64 is properly positioned in the patient and the split ball 76 is positioned onto the connection portion 60, the nut 80 receives the connection portion 60 as shown. Note that the nut 80 comprises an internal wall 90 that defines an internal bore 92 for receiving the connection portion 60. Note also that the nut 80 has an interior spherical wall 94 (Fig. 8B) that is adapted and dimensioned to complement the shape of the split ball 76 as best illustrated in Fig. 8B.

In the illustration being described, after the screw element 56 is screwed into bone, the bore 86 of fixation component 64 is received on the connection portion 60 along with the split ball 76. The connection portion 60 is also received in the internal bore 92 of the nut 80, and as the nut 80 is screwed onto the fixation component 64, as best illustrated in Fig. 8B, the interior spherical wall 94 cooperates with the spherical wall 70 of the fixation component 64 to compress the split ball 76 which causes it to compress against the connection portion 60, thereby locking the fixation component 64 to the screw element 56.

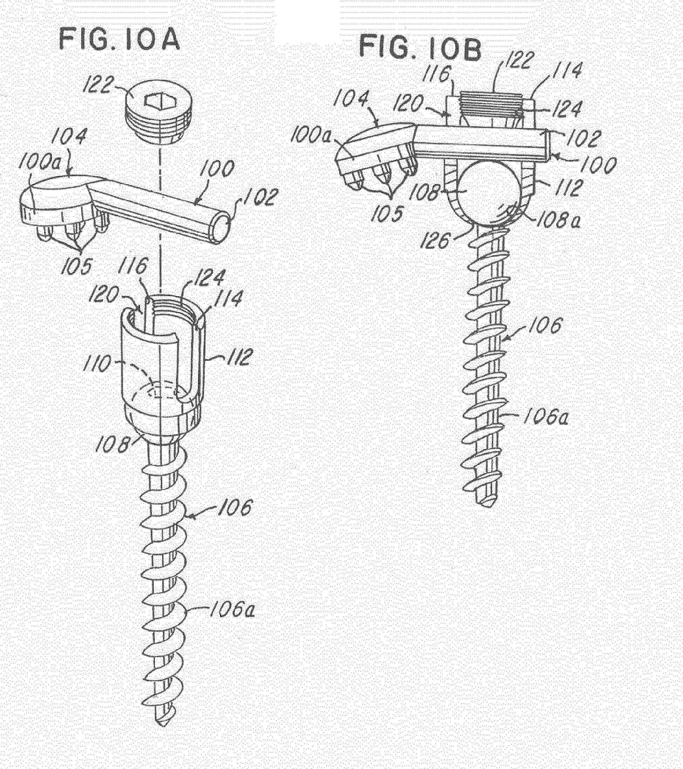

Of course, other means may be provided for locking the fixation component 20 and 64 onto the screw elements 16 and 56, respectively. For example, Fig. 10A illustrates another embodiment wherein a fixation component 100 has an engagement portion 104 that engages the at least one of the external facets or laminar surfaces of the second vertebra 14. In this regard, note that the embodiment comprises a polyaxial screw 106 having a spherical or polyaxial head 108 and a hex-female slot 110 for receiving a tool (not shown) for rotatably driving the polyaxial screw 106.

As illustrated in Fig. 10A, the polyaxial screw 106 comprises the spherical or polyaxial head 108 that is adapted to be received in a receiver 112 having a pair of walls 114 and 116 that define a channel 120. In this regard, the receiver 112 may comprise features illustrated in U.S. Patents 7,717,943; 7,662,172; 7,604,655; 7,686,835 and U.S. Patent Publication Nos. 2010/0204738; 2006/0155278; 2007/0123862; 2007/0093827;2007/0123867;2008/0071277;2008/0097457;2008/0249576; 2010/0063553; 2010/0179603 and 2010/0152788, all of which are incorporated herein by reference and made a part hereof.

Note in the illustration shown in Fig. 10A that the channel 120 receives an elongated rod or cylindrical portion 102 of the fixation component 100. The embodiment illustrated in Figs. 10A-10B comprises a threaded cap 122 that is threadably received in the female threads 124 in the receiver 112. The receiver 112 comprises a wall 126 (Fig. 10B) that defines a bore 128 for receiving the threaded shank 106a of the polyaxial screw 106.

After the surgeon screws the polyaxial screw 106 into bone, such as the first vertebra 12 (Fig. 11), the receiver 112 (Fig. 10A) is polyaxially positioned and the cylindrical portion 102 of the fixation component 100 is situated in the channel 120 and positioned such that the plurality of teeth 105 on end 100a engage at least one of the facet or laminar surface 14a of the second vertebra 14. Thereafter, the threaded cap 122 is threadably received in the female threads 124 of the receiver 112 and screwed thereon until the cylindrical portion 102 engages the polyaxial head 108 of the polyaxial screw 106, thereby locking the fixation component 100 into the receiver 112 and on the polyaxial screw 106 and fixing the facet joint associated with the first and second vertebrae 12 and 14. Although not shown, an external, rather than internal, cap could be used in this embodiment.

Fig. 10C illustrates still another embodiment of a polyaxial implant 130. In this embodiment, those parts that are the same or similar to the parts of the embodiments in Fig. 10A-10B are identified with the same part number, except that a prime mark ("'") has been added to the part numbers of the embodiment in Fig. 10C. Note in this embodiment that the locking is accomplished utilizing a receiver 132 having a bayonet-type connection. In this regard, note that the receiver 132 comprises walls 134 and 136 that define two generally L-shaped channels 138 and 140, respectively. In this embodiment, the receiver 132 receives the polyaxial screw 106' and the surgeon screws the polyaxial screw 106' into the first vertebra 12. The surgeon polyaxially moves the receiver 132 relative to the polyaxial head 108' until the fixation component 100' is situated in the proper position such that the plurality of teeth 105' are in engagement with the facet. Thereafter, the surgeon rotates the receiver 132 relative to the polyaxial screw 106' and fixation component 100' which causes the cylindrical portion 102' to be driven downward (as viewed in Fig. 10C) and against the head 108' of the polyaxial screw 106'. In this regard, the operation of the capless embodiments shown in Fig. 10C is substantially the same or similar to and may include the same features as the devices shown in U.S. Patents 7,717,943; 7,662,172; 7,604,655; 7,686,835 and U.S. Patent Publication Nos. 2010/0204738;2006/0155278;2007/0123862;2007/0093827;2007/0123867; 2008/0071277;2008/0097457;2008/0249576;2010/0063553;2010/0179603 and 2010/0152788, all of which are incorporated herein by reference and made a part hereof.

It should be understood that the embodiments shown in Fig. 10A-10C illustrate receivers 112 and 132 wherein the polyaxial screw 106 and 106' is loaded from the top (as viewed in Figs. 10A-10C) such that the polyaxial head 108 is seated against the spherical wall 108a (Fig. 10B). It should be understood, however, that the screw may be "bottom loaded" and similar to one or more of the devices shown in one or more of the above-referenced patents or publications earlier herein.

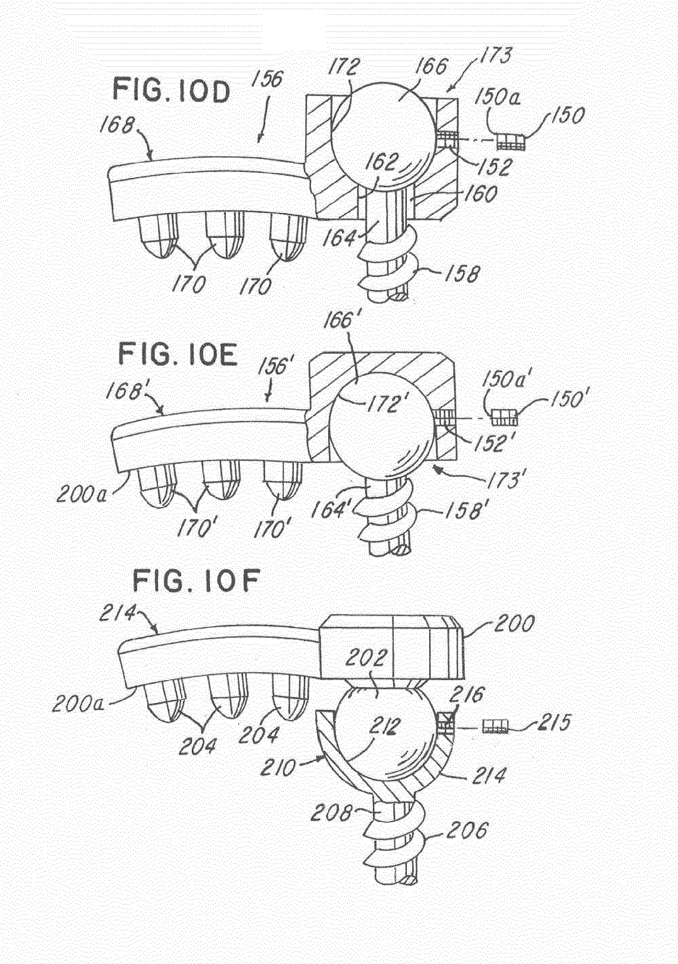

Fig. 10D illustrates yet another embodiment showing a fixation component 168 similar to the embodiment of Figs. 8A, 8B and 9 except that rather than a nut 80, a set screw 150 is used to lock the fixation component 168 to a polyaxial screw 158. This embodiment is similar to that shown in Fig. 9, except that the fixation component 168 does not utilize the nut 80 in cooperation with the male thread 78. In this regard, the implant 156 in Fig. 10D comprises a polyaxial screw 158 that is received in a bore 160 defined by an internal wall 162 that receives the shank 164 of the polyaxial screw 158. Note that the polyaxial screw 158 comprises the generally cylindrical head 166. The implant 156 comprises a fixation component 168 having the plurality of teeth 170 and a spherical shaped wall 172 that defines a socket 173 that receives the generally cylindrical head 166 as shown. After the surgeon screws the polyaxial screw 158 into the first vertebra 12, the fixation component 168 is polyaxially moved or situated against at least one of the external facet or laminar surface of the second vertebra 14. After the fixation component 168 is positioned where desired, the set screw 150 is screwed into the threaded aperture 152 until an end 150a engages the cylindrical head 166 of the polyaxial screw 158, thereby locking the fixation component 168 to the polyaxial screw 158.

The embodiment shown in Fig. 10E, with the like parts being identified with like part numbers except a prime mark ("'") is added to those in Fig. 10E, a "bottom-loaded" fixation component 168', rather than the top-loaded component 168 shown in Fig. 10D.

Fig. 10F illustrates still another embodiment of a ball and socket arrangement wherein the ball is located on or integral with the fixation component and the socket is situated in the screw head of a polyaxial screw. Like the embodiment illustrated in Fig. 10D, this embodiment permits polyaxial movement of the fixation component relative to the screw head.

In this embodiment, a fixation component 200 comprises an integral ball 202 and, like the prior embodiments, has a fixation surface 200a having a plurality of engagement teeth 204 as shown. The spinal implant in the embodiment of Fig. 10F further comprises a polyaxial screw 206 having a threaded shank 208 and a head portion 210. Note that the head portion 210 comprises an inner spherical surface 212 that defines a receiving area or socket 214 for receiving the ball 202. A set screw 215 is threadably screwed into the wall in the head 216 in order to lock the fixation component 200 to the screw 206.

In the illustrations shown in Figs. 10D, 10E and 10F, a ball and socket arrangement is used with a set screw, such as set screw 150, to lock the fixation component relative to the screw element. However, it should be understood that other means or apparatus could be used for locking these components together, such as providing an interference or frictional fit between the two elements; provided, however, that the components do not move relative to each other after placement in the patient.

Advantageously, the embodiments being described provide a surgical implant having an anatomically-shaped fixation element which on one end is secured to the screw element and on the other rigidly attaches to the external, non-articular, surface of the facet joint of the adjacent vertebra, such as the second vertebra 14. The embodiments described enable the implants to comprise means of attachment between the screw element and the fixation element and which provide the ability to vary the angles between the screw element and the fixation components to conform to regional anatomy of the patient.

While the system, apparatus and method herein described constitute preferred embodiments of this invention, it is to be understood that the invention is not limited to this precise system, apparatus and method, and that changes may be made therein without departing from the scope of the invention which is defined in the appended claims.

## Claims

1. A surgical implant for support of spinal vertebrae, said surgical implant comprising a screw element having a threaded shank for screwing into a pedicle of a first vertebrae and a fixation component that is moveably secured to said screw element, wherein said fixation component has a first end secured to said screw element and a fixation surface adapted to engage or attach to at least one of an external facet or laminar surface of a second vertebra, **characterized in that** said fixation component comprises a socket or ball and said screw element comprises a ball or socket, respectively, said ball being received in said socket to allow at least one of a polyaxial motion of said fixation component relative to said screw element or a polyaxial motion of said screw element relative to said fixation component.

2. The surgical implant according to claim 1, **characterized in that** said ball is located or integral with said screw element and said screw element comprises a receiver into which the ball is received, said receiver having a channel and said fixation element having an elongated member adapted to be received in said channel.

3. The surgical implant according to claim 2, **characterized in that** said elongated member is a rod extending from said first end of said fixation component.

4. The surgical implant according to any of claims 2 or 3, **characterized in that** said channel defines a bayonet channel for capturing and locking said elongated member therein upon rotation of said receiver.

5. The surgical implant according to any of claims 2 or 3, **characterized in that** said receiver comprises a cap for mounting on said receiver in order to block said elongated member in said receiver upon rotation of said receiver.

6. The surgical implant according to claim 1, **characterized in that** said screw element comprises a polyaxial screw having a polyaxial head, said fixation component comprising a socket for receiving said polyaxial head and at least one screw or cap for locking said fixation component to said polyaxial screw.

7. The surgical implant according to claim 1, **characterized in that** said screw element comprises a head having a socket and said fixation component comprises a ball or spherical component for mounting in said socket, said surgical implant further comprising a lock for locking said ball in said socket.

8. The surgical implant according to claim 7, **characterized in that** said lock is at least one of a set screw, a cap or an interference or frictional fit between said ball and said socket.

9. The surgical implant according to claim 8, **characterized in that** said fixation component has a bone-engagement portion and a locking portion for locking onto said screw element, said bone-engagement portion having an engagement surface for engaging bone, said bone-engagement portion being generally circular or elongated and generally conforming to a local anatomy.
